# EUROPEAN PATENT SPECIFICATION

(11) **EP 0 965 382 B1**
(45) Date of publication and mention of the grant of the patent: **03.09.2003**
(21) Application number: 98310372.2
(22) Date of filing: 17.12.1998
(51) Int. Cl.: B01J 23/34, B01J 23/26, B01J 23/745, C07C 41/03

(54) **Method for producing alkylene oxide adduct**
Verfahren zur Herstellung von Alkylenoxidaddukte
Procédé de préparation de produits d'addition d'oxyde d'alkylène

(30) Priority: 10.06.1998 JP 16171898
(43) Date of publication of application: 22.12.1999
(73) Proprietor: Lion Corporation, Tokyo 130-0004 (JP)
(72) Inventor: Okamoto, Takahiro, Soka-shi, Saitama 340-0031 (JP); Hama, Itsuo, Chiba-shi, Chiba 262-0032 (JP); Uemura, Shingo, Kodaira-shi, Tokyo 187-0003 (JP)
(74) Representative: Golding, Louise Ann

(56) References cited:
- EP-A- 0 689 870
- GB-A- 2 053 713
- US-A- 4 067 924
- US-A- 5 146 020
- US-A- 5 371 306
- US-A- 5 750 796
- WANG J ET AL: "A study of surface and inner layer compositions of Mg-Fe-Al-O mixed spinel sulfur-transfer catalyst using Auger electron spectroscopy" MATERIALS LETTERS, vol. 32, no. 4, 1 September 1997 (1997-09-01), page 223-227 XP004091490 ISSN: 0167-577X

## Description

The present invention relates to the use of an alkoxylation catalyst for producing an alkylene oxide adduct. More particularly, the invention relates to the use of an alkoxylation solid catalyst comprising a metal oxide, and to a method for producing an alkylene oxide adduct that is useful as a chemical material for a surfactant or the like.

A compound in which an alkylene oxide is added to an organic compound having an active hydrogen or to an ester is widely used as a chemical material for surfactants, solvents, or the like. Particularly, those obtained by polyalkoxylating alcohol, fatty acid, fatty acid ester, amine, alkylphenol, or the like with an alkylene oxide such as ethylene oxide or propylene oxide have been utilized as nonionic surfactants in a wide range of application.

As such an alkylene oxide adduct, one having a narrow adduct distribution has many advantages, e.g. high foamability, as compared with one having a wide adduct distribution. As a method for obtaining an alkylene oxide adduct having a narrow adduct distribution, those using a halide catalyst such as a halide of boron, tin, antimony, iron, or aluminum, or an acid catalyst such as phosphoric acid or sulfuric acid are well known. However, in such a method using an acid catalyst, sufficiently narrow adduct distribution cannot be obtained, and a large amount of by-product such as dioxane, dioxolane, or polyethylene glycol is produced. In addition, such an acid catalyst strongly corrodes materials of equipment.

Accordingly, as a solid catalyst for producing an alkylene oxide adduct having a narrow adduct distribution, the following composite oxides have been proposed.
1) Japanese Published Unexamined Patent Application No. (Tokkai hei) 1-164437: A method for producing an alkylene oxide adduct having a narrow adduct distribution uses as a catalyst a magnesium oxide in which a metal ion such as aluminum is added. It discloses, for example, a magnesium oxide catalyst containing 3 wt. % of aluminum.
2) Japanese Published Unexamined Patent Application No. (Tokkai hei) 2-71841: A method for producing an alkylene oxide adduct having a narrow adduct distribution with a calcined hydrotalcite as a catalyst is disclosed. The calcined hydrotalcite can be obtained by calcining a natural or synthetic hydrotalcite.
3) Japanese Published Unexamined Patent Application No. (Tokkai hei) 7-227540: A method for producing an alkylene oxide adduct with a magnesium oxide containing zinc, antimony, tin, or the like as a catalyst, in which generation of a by-product (polyethylene glycol) is inhibited, is disclosed. By using the Mg-Zn, Mg-Sb or Mg-Sn composite oxide catalyst, the amount of polyethylene glycol formed as a by-product can be reduced, although the catalytic activity may be decreased as compared with a case using a magnesium oxide catalyst in which aluminum is added. However, the effect of inhibiting polyethylene glycol formation is still insufficient.
4) Japanese Published Unexamined Patent Application No. (Tokkai hei) 8-268919: A method for producing an alkylene oxide adduct having a narrow adduct distribution uses as a catalyst an Al-Mg composite oxide which is obtained by calcining aluminum magnesium hydroxide.

An alkylene oxide adduct obtained using each of the above-mentioned catalysts has a narrower adduct distribution than that obtained with an acid catalyst. Moreover, generation of a by-product such as dioxane can be inhibited. Particularly, a composite oxide of magnesium and aluminum has a high activity. However, the composite oxide catalyst cannot inhibit formation of polyalkylene glycol as a by-product. Japanese Published Unexamined Patent Application No. (Tokkai hei) 7-227540 discloses a catalyst capable of reducing the amount of polyalkylene glycol formed as a by-product. However, the effect of inhibiting polyethylene glycol formation is still insufficient. Moreover, it is a high molecular weight polyalkylene glycol with a molecular weight of several tens of thousands that causes particularly difficult problems. Even a trace of high molecular weight polyalkylene glycol can cause problems in polyalkoxylating. For example, removing catalysts may become difficult, and the stability of a product containing the alkylene oxide adduct may be reduced. Moreover, a catalyst for producing an alkylene oxide adduct is required to have a sufficient catalytic activity in practical use.

Accordingly, it is an object of the present invention to provide a method for producing an alkylene oxide adduct having a narrow adduct distribution using a catalyst. Particularly, it is an object of the present invention to provide a catalyst with which an alkylene oxide adduct having a narrow adduct distribution can be produced efficiently while inhibiting formation of high molecular weight polyalkylene glycol.

As a result of earnest research with respect to an alkoxylation catalyst suitable for production of an alkylene oxide adduct having a narrow adduct distribution, the inventors have found that it is possible to achieve both high catalytic activity and inhibition of high molecular weight polyalkylene glycol formation with use of a catalyst prepared by adding a particular metal to a Mg-Al composite oxide.

The above-mentioned metal added to the Mg-Al composite oxide is selected from chromium, manganese and iron.

The alkoxylation catalyst for use in the present invention is obtained by adding the metal as a third component to Mg-Al composite oxide containing simultaneously a basic site of an oxygen atom adjacent to a magnesium atom for activating an organic compound having an active hydrogen, and an acidic site of an aluminum atom for activating an alkylene oxide. Mg-Al composite oxide has been conventionally utilized, and it is a highly active catalyst with which an alkylene oxide adduct having a narrow adduct distribution can be produced. Moreover, according to the present invention, formation of high molecular weight polyalkylene oxide glycol as a by-product can be inhibited by the third component. It is believed that the addition of the metal as a third component causes a structural change in the active site of the side reaction.

Thus, by using the method of the present invention, it is possible to produce an alkylene oxide adduct having a narrow adduct distribution efficiently, while inhibiting formation of high molecular weight polyalkylene glycol as a by-product. Furthermore, the method for producing an alkylene oxide adduct according to the present invention comprises adding an alkylene oxide to an organic compound in the presence of the alkoxylation catalyst.

The present invention will be described below in detail referring to the drawings, in which:
Figure 1 is a graph showing the adduct distribution of an EO adduct obtained according to Reaction Example 1 using the method of the present invention;
Figure 2 is a graph showing the adduct distribution of an EO adduct obtained according to Reaction Example 2 using the method of the present invention;
Figure 3 is a graph showing the adduct distribution of an EO adduct obtained according to Reaction Example 3 using the method of the present invention;
Figure 4 is a graph showing the adduct distribution of an EO adduct obtained according to Reaction Example 4 using the method of the present invention;
Figure 5 is a graph showing the adduct distribution of an EO adduct obtained according to Reaction Example 5 using the method of the present invention;
Figure 6 is a graph showing the adduct distribution of an EO adduct obtained according to Reaction Example 6 using the method of the present invention;
Figure 7 is a graph showing the adduct distribution of an EO adduct obtained according to Comparative Reaction Example 1;
Figure 8 is a graph showing the adduct distribution of an EO adduct obtained according to Comparative Reaction Example 2;
Figure 9 is a graph showing the adduct distribution of an EO adduct obtained according to Comparative Reaction Example 3;
Figure 10 is a graph showing the adduct distribution of an EO adduct obtained according to Reaction Example 11 using the method of the present invention;
Figure 11 is a graph showing the adduct distribution of an EO adduct obtained according to Comparative Reaction Example 4; and
Figure 12 is a graph showing the adduct distribution of an EO adduct obtained according to a reaction example using a KOH catalyst.

The catalyst for use in the present invention will be described in detail as follows.

As the metal of the third component in the catalyst of the present invention, chromium, manganese, or iron is used. More preferably, manganese is used. A combination of at least two of the above-mentioned metals may be also used as the third component.

In the catalyst for use in the present invention, it is preferable that the ratio of each metal is within a range suitable for inhibiting formation of high molecular weight polyalkylene glycol as a by-product, while maintaining high catalytic activity of Mg-Al composite oxide. A preferable ratio of each metal is described below.

The atomic ratio between magnesium and aluminum as shown by Al/(Mg+Al) is preferably in the range of 0.1 to 0.7, more preferably 0.3 to 0.6.

The atomic ratio of the metal added as a third component with respect to the total metals is preferably in the range of 0.05 to 0.4, more preferably 0.1 to 0.25. If the amount of the third component is too small, the effect of inhibiting formation of high molecular weight polyalkylene glycol cannot be obtained sufficiently. On the other hand, if the amount of the third component is excessively large, the catalytic activity may be decreased.

The catalyst for use in the present invention can be obtained by a known method for preparing a multi-element composite oxide, for example, by the impregnation method or coprecipitation method. A process of preparing the catalyst for use in the present invention by the coprecipitation method is herein described.

According to the coprecipitation method, first a mixed aqueous solution containing a metal compound such as nitrate, sulfate, carbonate, acetate, or chloride of each metal is prepared, and a precipitant is added to the aqueous solution. The precipitate obtained by the addition of the precipitant is treated by washing with water, drying, calcining, or the like so as to form a catalyst comprising a composite oxide.

A particular example applying the coprecipitation method to the catalyst for use in present invention is a method of forming a precipitate by dropping a mixed aqueous solution containing metal compounds of magnesium, aluminum and a metal as a third component with a mixed solution as a precipitant (e.g. a mixed aqueous solution of sodium hydroxide and sodium carbonate) at a predetermined flow amount, while maintaining a predetermined pH. In this example, it is preferable that the pH of the solution is adjusted within the range of 7 to 11, preferably 8 to 10. From the composite hydroxide obtained, water soluble salts are removed by washing with water, and then it is dried. Thereafter, it is calcined by heating at 300 to 1000 °C, preferably at 600 to 900 °C. Thus, a catalyst for use in the present invention comprising a composite oxide can be obtained.

Next, an organic compound to which alkylene oxide is added using the method of the present invention will be described. Such an organic compound is not particularly limited, as long as it can be alkoxylated, but particularly used is an organic compound having an active hydrogen or an ester. More particularly, alcohols, phenols, fatty acids, fatty acid esters, fatty amines, fatty acid amides, polyols, or a mixture thereof are suitably used. Typical examples of them are illustrated in the following.

As alcohols, it is preferable to use a saturated or unsaturated primary or secondary alcohol having 2 to 30 carbon atoms, more preferably a primary alcohol having 4 to 24 carbon atoms, particularly preferably a primary alcohol having 6 to 24 carbon atoms. Furthermore, as phenols, it is preferable to use mono-, di-, or trialkylphenol, particularly a compound having 4 to 12 carbon atoms in an alkyl group.

As fatty acids, a fatty acid having 8 to 22 carbon atoms, for example, a saturated or unsaturated straight-chain fatty acid obtainable by fat decomposition of coconut oil, palm oil, palm kernel oil, soybean oil, sunflower oil, rapeseed oil, fish fat, or the like (e.g. caprylic acid, n-capric acid, lauric acid, myristic acid, oleic acid, or stearic acid), or a mixture thereof preferably can be used. Furthermore, as fatty acid esters, it is preferable to use those produced by esterifying the above-mentioned fatty acids with an alcohol of alkyl group having 1 to 4 carbon atoms.

As fatty amines, it is preferable to use a primary fatty amine obtainable from a saturated or unsaturated straight-chain fatty acid or a compound in which nitrile is introduced into corresponding aliphatic alcohol. Furthermore, as the fatty acid amides, it is preferable to use a derivative obtainable by reaction between a saturated or unsaturated straight-chain fatty acid and ammonia or primary fatty amine.

As the polyols, it is preferable to use polyethylene glycol or polypropylene glycol having an average degree of polymerization of 2 to 2,000, or glycerol, sorbitol, or the like.

On the other hand, in the method of the present invention, it is preferable to use an alkylene oxide having 2 to 8 carbon atoms, particularly preferably ethylene oxide, propylene oxide, or butylene oxide having 2 to 4 carbon atoms.

In the following, preferable conditions of reaction in the method for producing an alkylene oxide adduct according to the present invention will be described. The reaction temperature is preferably 80 to 230 °C, more preferably 120 to 200 °C, most preferably 160 to 180 °C. Although the reaction pressure also depends on the reaction temperature, it is preferably 0 to 20 atm, more preferably 2 to 8 atm. Although it also depends on the molar ratio of the alkylene oxide and the starting material provided in the reaction, usually the amount of the catalyst used is preferably 0.01 to 20 wt. %, more preferably 0.05 to 5 wt. %, of the starting material such as alcohoL

The reaction operation is as follows. For example, starting material such as alcohol and a catalyst are put in an autoclave. After the substitution of nitrogen gas for the air in the autoclave, an alkylene oxide is introduced into the autoclave to cause reaction under predetermined temperature and pressure conditions. The catalyst may be present in the reaction product depending on its use, however, it is usually separated from the reaction product by filtering, which is performed after adding water or a filter aid to decrease viscosity.

According to the method of the present invention, an alkylene oxide adduct having a very narrow adduct distribution can be produced more efficiently with high catalytic activity, with a very small amount of high molecular weight polyethylene glycol formed as a by-product. Particularly, because formation of high molecular weight polyalkylene glycol as a by-product is inhibited, the efficiency of filtering catalyst can be improved, and also the properties (e.g. stability in low temperature) of a chemical product using the obtained alkylene oxide adduct as a material are improved.

The present invention will be described below in more detail by way of examples and comparative examples. These examples are illustrative in nature and should not be considered as limiting the present invention.

### Example 1

To prepare a solution A, 68.03 g (0.265 mol) of magnesium nitrate hexahydrate, 47.69 g (0.127 mol) of aluminum nitrate nonahydrate, and 24.33 g (0.085 mol) of manganese nitrate hexahydrate were dissolved in 450 g of deionized water. On the other hand, 13.47 g (0.127 mol) of sodium carbonate was dissolved in 450 g of deionized water to prepare a solution B.

The solutions A and B were dropped into a catalyst preparation vessel previously supplied with 1,800 g of deionized water over a period of 1 hour, while maintaining the pH of 9 with 2N-NaOH and the temperature of 40 °C. After completing the dropping, the mixed solution was aged for 1 hour. The mother liquor was removed by filtration, and the precipitate was washed with 6 liters of deionized water and spray-dried, and 30 g of a composite hydroxide was obtained. The composite hydroxide was calcined for 3 hours at 800 °C in a nitrogen atmosphere to obtain 19 g of a Mg-Al-Mn composite oxide catalyst (Mg : Al : Mn (by atomic ratio) = 0.56: 0.26 : 0.18).

### Example 2

To prepare a solution A, 68.03 g (0.265 mol) of magnesium nitrate hexahydrate, 47.69 g (0.127 mol) of aluminum nitrate nonahydrate, and 20.83 g (0.085 mol) of manganese acetate tetrahydrate were dissolved in 450 g of deionized water. On the other hand, 13.47 g (0.127 mol) of sodium carbonate was dissolved in 450 g of deionized water to prepare a solution B.

After that, according to the same procedure as in Example 1, 19 g of a Mg-Al-Mn composite oxide catalyst (Mg : Al : Mn (by atomic ratio) = 0.56 : 0.26 : 0.18) was obtained.

### Example 3

To prepare a solution A, 69.68 g (0.272 mol) of magnesium nitrate hexahydrate, 61.06 g (0.163 mol) of aluminum nitrate nonahydrate, and 14.60 g (0.051 mol) of manganese nitrate hexahydrate were dissolved in 450 g of deionized water. On the other hand, 17.26 g (0.163 mol) of sodium carbonate was dissolved in 450 g of deionized water to prepare a solution B.

After that, according to the same procedure as in Example 1, 21 g of a Mg-Al-Mn composite oxide catalyst (Mg : Al : Mn (by atomic ratio) = 0.56 : 0.34 : 0.10) was obtained.

### Example 4

To prepare a solution A, 66.72 g (0.260 mol) of magnesium nitrate hexahydrate, 38.98 g (0.104 mol) of aluminum nitrate nonahydrate, and 29.82 g (0.104 mol) of manganese nitrate hexahydrate were dissolved in 450 g of deionized water. On the other hand, 11.01 g (0.104 mol) of sodium carbonate was dissolved in 450 g of deionized water to prepare a solution B.

After that, according to the same procedure as in Example 1, 18 g of a Mg-Al-Mn composite oxide catalyst (Mg : Al : Mn (by atomic ratio) = 0.56 : 0.22 : 0.22) was obtained.

### Example 5

To prepare a solution A, 57.26 g (0.223 mol) of magnesium nitrate hexahydrate, 50.06 g (0.133 mol) of aluminum nitrate nonahydrate, and 35.60 g (0.089 mol) of chromium nitrate nonahydrate were dissolved in 450 g of deionized water. On the other hand, 23.57 g (0.222 mol) of sodium carbonate was dissolved in 450 g of deionized water to prepare a solution B.

After that, according to the same procedure as in Example 1, 23 g of a Mg-Al-Cr. composite oxide catalyst (Mg : Al : Cr (by atomic ratio) = 0.50 : 0.30 : 0.20) was obtained.

### Example 6

To prepare a solution A, 68.03 g (0.265 mol) of magnesium nitrate hexahydrate, 47.69 g (0.127 mol) of aluminum nitrate nonahydrate, and 21.35 g (0.085 mol) of ferrous chloride tetrahydrate were dissolved in 450 g of deionized water. On the other hand, 13.47 g (0.127 mol) of sodium carbonate was dissolved in 450 g of deionized water to prepare a solution B.

After that, according to the same procedure as in Example 1, 18 g of a Mg-Al-Fe composite oxide catalyst (Mg : Al : Fe (by atomic ratio) = 0.56 : 0.26 : 0.18) was obtained.

### Example 7

Following the same procedure as in Example 1 except that 16.82 g (0.085 mol) of manganese chloride tetrahydrate was used as the manganese salt, 19 g of a Mg-Al-Mn composite oxide catalyst (Mg : Al : Mn (by atomic ratio) = 0.56 : 0.26 : 0.18) was obtained.

### Example 8

Following the same procedure as in Example 1 except that 20.49 g (0.085 mol) of manganese sulfate pentahydrate was used as the manganese salt, 19 g of a Mg-Al-Mn composite oxide catalyst (Mg : Al : Mn (by atomic ratio) = 0.56 : 0.26 : 0.18) was obtained.

### Example 9

Following the same procedure as in Example 1 except that 9.77 g (0.085 mol) of manganese carbonate was used as the manganese salt, 19 g of a Mg-Al-Mn composite oxide catalyst (Mg : Al : Mn (by atomic ratio) = 0.56 : 0.26 : 0.18) was obtained.

### Example 10

To prepare a solution A, 317.48 g (1.24 mol) of magnesium nitrate hexahydrate, 222.55 g (0.593 mol) of aluminum nitrate nonahydrate, and 96.9 g (0.396 mol) of manganese acetate tetrahydrate were dissolved in 600 g of deionized water. On the other hand, 62.88 g (0.593 mol) of sodium carbonate was dissolved in 500 g of deionized water to prepare a solution B.

The solutions A and B were dropped into a catalyst preparation vessel previously supplied with 480 g of deionized water over a period of 1 hour, while maintaining the pH of 9 with 10N-NaOH and the temperature of 70 °C. After completing the dropping step, the mixture solution was aged for 1 hour. The mother liquor was removed by filtration, and the precipitate was washed with 9.6 liters of deionized water and spray-dried, and 92 g of a composite hydroxide was obtained. The composite hydroxide was calcined for 3 hours at 800 °C in a nitrogen atmosphere to obtain 60 g of a Mg-Al-Mn composite oxide catalyst (Mg : Al : Mn (by atomic ratio) = 0.56 : 0.26 : 0.18).

### Comparative Example 1

To prepare a solution A, 57.26 g (0.223 mol) of magnesium nitrate hexahydrate and 66.80 g (0.178 mol) of aluminum nitrate nonahydrate were dissolved in 450 g of deionized water. On the other hand, 18.87 g (0.178 mol) of sodium carbonate was dissolved in 450 g of deionized water to prepare a solution B.

After that, following the same procedure as in Example 1, 17 g of a Mg-Al composite oxide catalyst (Mg : Al (by atomic ratio) = 0.556 : 0.444) was obtained.

### Comparative Example 2

25 g of aluminum magnesium hydroxide with a chemical composition of 2.5MgO·Al₂O₃·mH₂O (KYOWAAD (registered trademark) 300 manufactured by Kyowa Chemical Industry) was calcined in a nitrogen atmosphere at 800 °C for 3 hours, and 16 g of Mg-Al composite oxide catalyst was obtained.

### Comparative Example 3

25 g of hydrotalcite with a chemical composition of Mg₆Al₂(OH)₁₆CO₃·4H₂O (KYOWAAD (registered trademark) 500 manufactured by Kyowa Chemical Industry) was calcined in a nitrogen atmosphere at 500 °C for 3 hours, and 18 g of Mg-Al composite oxide catalyst was obtained.

Using each catalyst obtained in Examples 1 to 10 and Comparative Examples 1 to 3, alkylene oxide addition reactions were carried out according to the reaction evaluation method I as described below. The reactions are referred to as Reaction Examples 1 to 10 and Comparative Reaction Example 1 to 3, respectively. Furthermore, the catalytic activity and the amount of high molecular weight polyethylene glycol (PEG) formed as a by-product in each reaction example were evaluated according to the respective methods as described below. The evaluation results are shown in Table 1.

Furthermore, using each catalyst obtained in Example 1 and Comparative Example 1, alkylene oxide addition reactions were carried out according to the reaction evaluation method II as described below. The reactions are referred to as Reaction Example 11 and Comparative Reaction Example 4, respectively. The evaluation results of the reactions are shown in Table 2.

### Reaction Evaluation Method I

400 g of lauryl alcohol and 0.4 g of a catalyst were put in a 4-liter capacity autoclave. The air in the autoclave was replaced with nitrogen gas, and the temperature was increased while stirring. Then, while maintaining the temperature of 180 °C and the pressure of 3 atm, 663 g of ethylene oxide (EO) (average adduct molar number: 7) was introduced to cause reaction between the lauryl alcohol and the EO.

### Reaction Evaluation Method II

400 g of methyl laurate, 1.2 g of catalyst, and 0.12 g of 40 % KOH aqueous solution were put in a 4 liter capacity autoclave. The air in the autoclave was replaced with nitrogen gas, and the temperature was increased while stirring. Then, while maintaining the temperature of 180 °C and the pressure of 3 atm. 494 g of EO (average adduct molar number: 6) was introduced to cause reaction between the methyl laurate and the EO.

### Catalytic Activity

In each of the above-mentioned reaction evaluation methods, the EO supplying rate (g-EO/min) was converted into a value per unit amount of catalyst after a point when the temperature and the pressure reached the predetermined values (180 °C and 3 atm, respectively). The obtained value was used as an evaluation measure of the catalytic activity (the unit: [g-EO/(min·g-catalyst)]). The EO supplying rate corresponds to the amount of EO consumed per unit time under the above-mentioned predetermined temperature and pressure conditions. During this measurement, the catalyst concentration was adjusted to a low level so that catalytic activity under the control of chemical reaction rate can be evaluated accurately.

### The Amount of High Molecular Weight PEG Formed as By-product

In each of the above reaction evaluation methods, the content of high molecular weight polyethylene glycol having a molecular weight of at least 20,000 in the reaction product was analyzed quantitatively according to the HPLC method, and the comparison was made based on weight %.

**Table 1**

| Reaction Examples 1 to 10 and Comparative Reaction Examples 1 to 3. | | | |
|---|---|---|---|
| | Composite Oxide Catalyst | Catalytic Activity (g-EO/(min·g-cat)) | Amount of High Molecular Weight PEG Formed as By-product (wt. %) |
| Reaction Example 1 | Catalyst of Ex. 1 | 6.0 | 0.06 |
| 2 | Catalyst of Ex. 2 | 11.1 | 0.04 |
| 3 | Catalyst of Ex. 3 | 8.6 | 0.09 |
| 4 | Catalyst of Ex. 4 | 3.4 | 0.05 |
| 5 | Catalyst of Ex. 5 | 3.6 | 0.16 |
| 6 | Catalyst of Ex. 6 | 3.0 | 0.20 |
| 7 | Catalyst of Ex. 7 | 6.1 | 0.05 |
| 8 | Catalyst of Ex. 8 | 4.5 | 0.02 |
| 9 | Catalyst of Ex. 9 | 4.7 | 0.05 |
| 10 | Catalyst of Ex. 10 | 9.2 | 0.04 |
| Comparative Reaction Example 1 | Catalyst of Comp. Ex. 1 | 5.7 | 0.53 |
| 2 | Catalyst of Comp. Ex. 2 | 4.8 | 0.60 |
| 3 | Catalyst of Comp. Ex. 3 | 6.7 | 1.10 |

**Table 2**

| Reaction Example 11 and Comparative Reaction Example 4. | | | |
|---|---|---|---|
| | Composite Oxide Catalyst | Catalytic Activity (g-EO/(min·g-cat)) | Amount of High Molecular Weight PEG Formed as By-product (wt. %) |
| Reaction Example 11 | Catalyst of Ex. 1 | 2.7 | 0.06 |
| Comparative Reaction Example 4 | Catalyst of Comp. Ex. 1 | 1.9 | 0.58 |

As is apparent from the results in Tables 1 and 2, formation of high molecular weight polyethylene glycol as a by-product is inhibited when using the method of the present invention. Particularly, when using the catalyst of Example 1 containing Mn, the amount of high molecular weight polyethylene glycol formed as a by-product is decreased to about one tenth the amount of that formed with the catalyst of Comparative Example 1, which was prepared according to the same procedure but not containing Mn.

Furthermore, it was confirmed that the ability of catalyst is influenced depending on the type of the starting manganese salt. For example, the acetate is preferable with respect to catalytic activity, and the sulfate is preferable for inhibiting formation of high molecular weight polyethylene glycol as a by-product.

In a multi-layered hydroxide as a catalyst precursor before being calcined, anions are believed to be taken in the guest layer having a layered structure (while the host layer is metal hydroxide). As a result, the anions influence the crystal structure of the catalyst precursor.

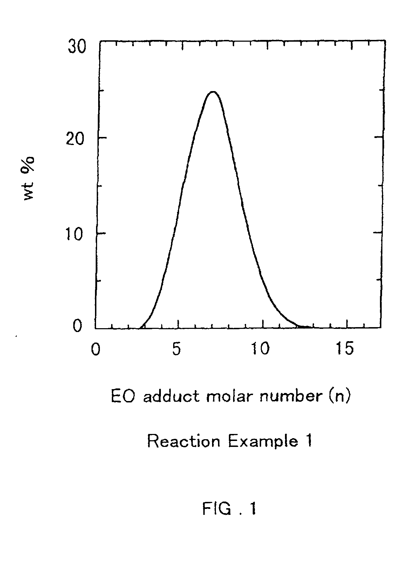

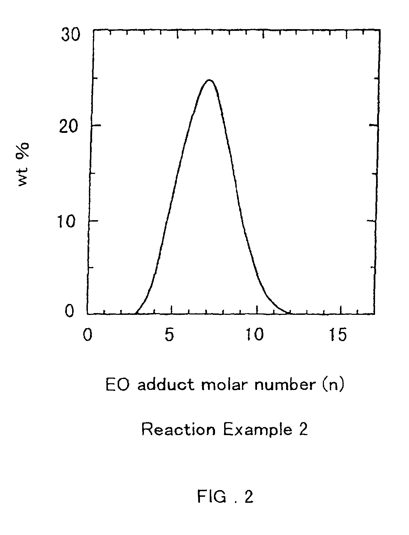

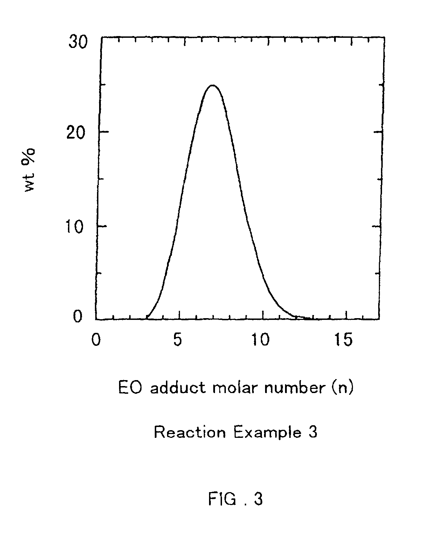

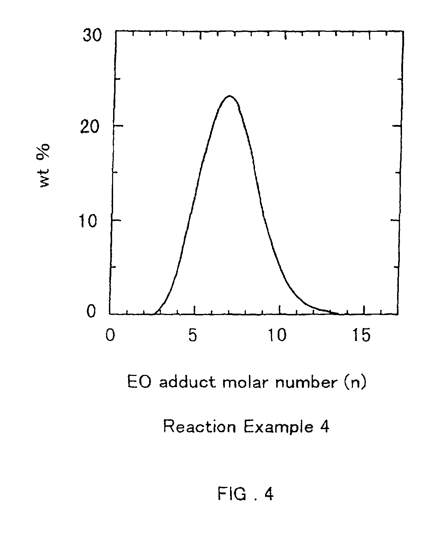

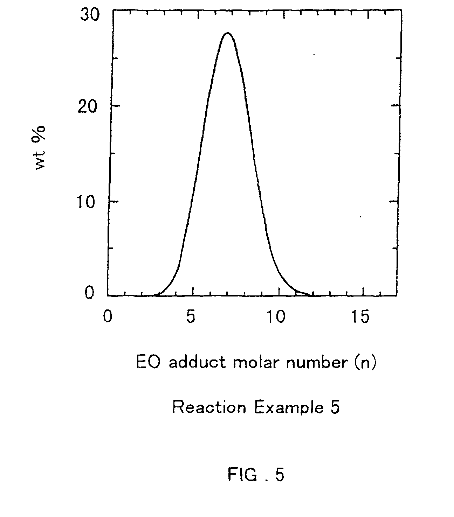

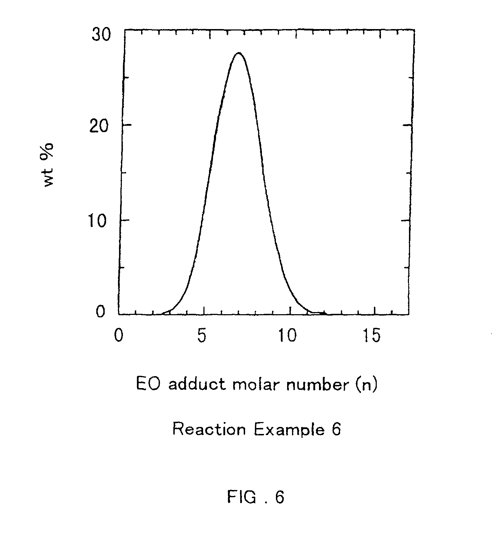

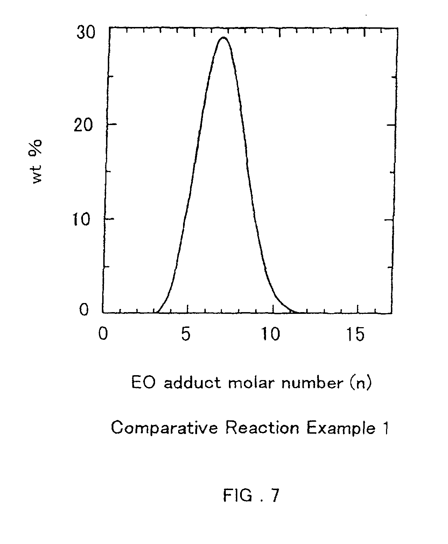

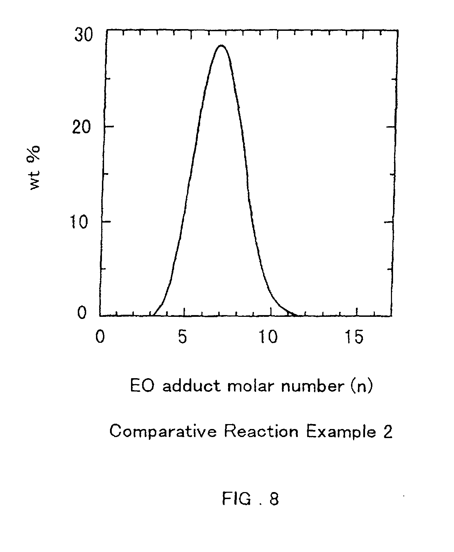

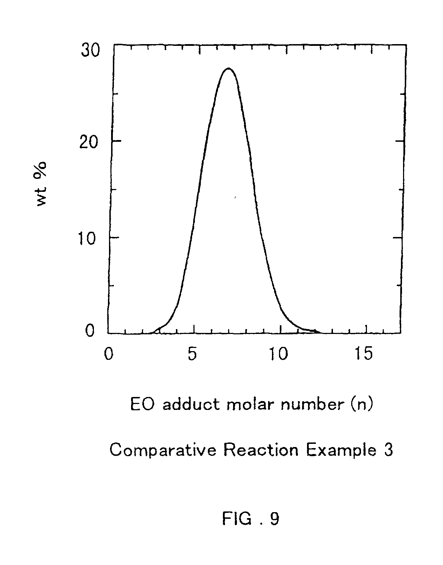

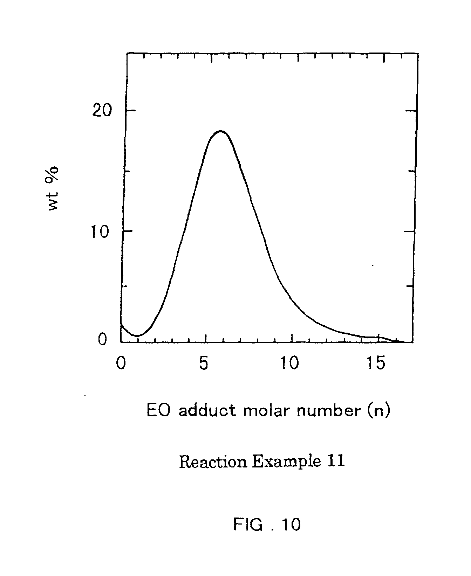

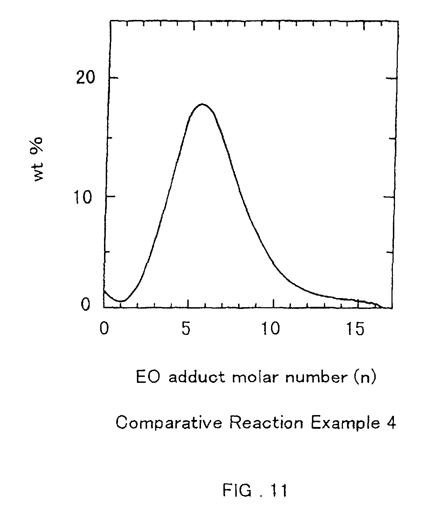

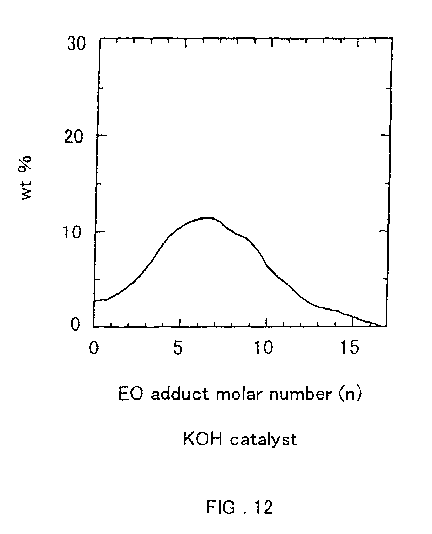

With respect to the EO adducts obtained in Reaction Examples 1 to 6 and 11 and Comparative Reaction Examples 1 to 4, EO adduct distribution was measured by the HPLC method. The results are shown in Figures 1 to 11. Furthermore, for the purpose of comparing the adduct distribution, alkylene oxide addition reaction was carried out using a KOH catalyst as an alkali catalyst according to the above-mentioned reaction evaluation method I. The adduct distribution of the EO adduct obtained by this reaction was measured in the same way as mentioned above. The result is shown in Figure 12.

## Claims

1. A method for producing an alkylene oxide adduct, which comprises adding an alkylene oxide to an organic compound in the presence of an alkoxylation catalyst, said catalyst comprising a metal oxide containing magnesium, aluminum, and at least one metal selected from chromium, manganese and iron.

2. The method as claimed in claim 1, wherein the organic compound is at least one selected from a group consisting of alcohols, phenols, fatty acids, fatty acid esters, fatty amines, fatty acid amides and polyols.

3. The method as claimed in claim 2, wherein the at least one organic compound is at least one alcohol selected from primary alcohols and secondary alcohols having 2 to 30 carbon atoms.

4. The method as claimed in claim 2, wherein the at least one organic compound is at least one primary alcohol having 6 to 24 carbon atoms.

5. The method as claimed in claim 2, wherein the at least one organic compound is at least one fatty acid ester of a fatty acid having 8 to 22 carbon atoms with an alkanol having 1 to 4 carbon atoms.

6. The method as claimed in any one of claims 1 to 5, wherein said catalyst has an atomic ratio between magnesium and aluminum as shown by Al/(Mg + Al) which is in the range of 0.1 to 0.7.

7. The method as claimed in claim 6, wherein said ratio is in the range of 0.3 to 0.6.

8. The method as claimed in any preceding claim, wherein said catalyst has an atomic ratio of the metals selected from chromium, manganese and iron which with respect to the total of magnesium, aluminum and said metals is in the range of 0.05 to 0.4.

## Patentansprüche

1. Verfahren zur Herstellung eines Alkylenoxid-Adduktes, umfassend die Zugabe eines Alkylenoxids zu einer organischen Verbindung in der Gegenwart eines Alkoxylierungskatalysators, wobei der Katalysator ein Metalloxid enthaltend Magnesium, Aluminium und zumindest ein Metall ausgesucht aus Chrom, Mangan und Eisen umfasst.

2. Verfahren gemäß Anspruch 1, wobei die organische Verbindung zumindest eine Verbindung ausgesucht aus der Gruppe bestehend aus Alkoholen, Phenolen, Fettsäuren, Fettsäureestern, Fettaminen, Fettsäureamiden und Polyolen ist .

3. Verfahren gemäß Anspruch 2, wobei die zumindest eine organische Verbindung zumindest ein Alkohol ausgesucht aus primären Alkoholen und sekundären Alkoholen mit 2 bis 30 Kohlenstoffatomen ist.

4. Verfahren gemäß Anspruch 2, wobei die zumindest eine organische Verbindung zumindest ein primärer Alkohol mit 6 bis 24 Kohlenstoffatomen ist.

5. Verfahren gemäß Anspruch 2, wobei die zumindest eine organische Verbindung zumindest ein Fettsäureester einer Fettsäure mit 8 bis 22 Kohlenstoffatomen mit einem Alkanol mit 1 bis 4 Kohlenstoffatomen ist.

6. Verfahren gemäß einem der vorherigen Ansprüche 1 bis 5, wobei der Katalysator ein Atomverhältnis von Magnesium zu Aluminium, wie durch Al/(Mg + Al) ausgedrückt, aufweist, das im Bereich von 0,1 bis 0,7 liegt.

7. Verfahren gemäß Anspruch 6, wobei das Verhältnis im Bereich von 0,3 bis 0,6 liegt.

8. Verfahren gemäß einem der vorherigen Ansprüche, wobei der Katalysator ein Atomverhältnis der Metalle ausgesucht aus Chrom, Mangan und Eisen aufweist, das im Verhältnis zu der Gesamtheit aus Magnesium, Aluminium und den oben genannten Metallen im Bereich von 0,05 bis 0,4 liegt.

## Revendications

1. Procédé pour produire un produit d'addition d'oxyde d'alkylène, qui comprend l'addition d'un oxyde d'alkylène à un composé organique en présence d'un catalyseur d'alcoxylation, ledit catalyseur comprenant un oxyde métallique contenant du magnésium, de l'aluminium et au moins un métal choisi parmi le chrome, le manganèse et le fer.

2. Procédé selon la revendication 1, dans lequel le composé organique est au moins un composé choisi dans le groupe constitué par les alcools, les phénols, les acides gras, les esters d'acide gras, les amines grasses, les amides d'acide gras et les polyols.

3. Procédé selon la revendication 2, dans lequel le au moins un composé organique est au moins un alcool choisi parmi les alcools primaires et les alcools secondaires ayant de 2 à 30 atomes de carbone.

4. Procédé selon la revendication 2, dans lequel le au moins un composé organique est au moins un alcool primaire ayant de 6 à 24 atomes de carbone.

5. Procédé selon la revendication 2, dans lequel le au moins un composé organique est au moins un ester d'acide gras d'un acide gras ayant de 8 à 22 atomes de carbone avec un alcanol ayant de 1 à 4 atomes de carbone.

6. Procédé selon l'une quelconque des revendications 1 à 5, dans lequel ledit catalyseur a un rapport atomique entre le magnésium et l'aluminium, tel qu'indiqué par Al/(Mg + Al) qui est situé dans la plage allant de 0,1 à 0,7.

7. Procédé selon la revendication 6, dans lequel ledit rapport est situé dans la plage allant de 0,3 à 0,6.

8. Procédé selon l'une quelconque des revendications précédentes, dans lequel ledit catalyseur a un rapport atomique des métaux choisis parmi le chrome, le manganèse et le fer, par rapport au total du magnésium, de l'aluminium et desdits métaux, qui est situé dans la plage allant de 0,05 à 0,4.
